**Europäisches Patentamt**

**European Patent Office**

**Office européen des brevets**

(19)

(11) Numéro de publication: **0 202 142**
**B1**

# (12) FASCICULE DE BREVET EUROPEEN

(45) Date de publication du fascicule du brevet:
**31.01.90**

(51) Int. Cl.⁴: **A61K 39/205, C12N 15/00**

(21) Numéro de dépôt: **86400780.2**

(22) Date de dépôt: **11.04.86**

(54) Vaccin antirabique avirulent.

(30) Priorité: **12.04.85 FR 8505575**

(43) Date de publication de la demande:
**20.11.86 Bulletin 86/47**

(45) Mention de la délivrance du brevet:
**31.01.90 Bulletin 90/5**

(84) Etats contractants désignés:
**AT BE CH DE FR GB IT LI LU NL SE**

(73) Titulaire: Etablissement Public dit: **CENTRE NATIONAL DE LA RECHERCHE SCIENTIFIQUE (CNRS), 15, Quai Anatole France, F-75007 Paris(FR)**

(72) Inventeur: **Flamand, Anne, 6 bis, Le Bois du Roi, F-91940 Les Ulis(FR)**
Inventeur: **Coulon, Patrice, 17, Allée de la Butte de Rheims, F-91120 Palaiseau(FR)**
Inventeur: **Prehaud, Christophe, 11, Rue Charles Péguy, F-78460 Chevreuse(FR)**

(74) Mandataire: **Gillard, Marie-Louise et al, Cabinet Beau de Loménie 55, Rue d'Amsterdam, F-75008 Paris(FR)**

(56) Documents cités: (suite)
BIOLOGICAL ABSTRACTS,
vol. 72, 1981, no. 45340, Biological Abstracts, Inc., Philadelphia, PA, US; M.F.A. AUBERT et al.: "Pathogenic, immunogenic and protective powers of 10 temperature-sensitive mutants of rabies virus in mice", & ANN. VIROL.
(PARIS) 1980, 131(2), 217-228OL. 1985, 53(3), 926-934s"idazines", & FARMACO, ED.
SCI. 1982, 37(2), 133-44 000
CHEMICAL ABSTRACTS,
vol.101, no. 17, 22 octobre 1984, page 529, no. 149417c, Columbus, Ohio, US; A. FLAMAND et al.: "Immunogenic and protective power of avirulent mutants of rabies virus selected with neutralizing monoclonal antibodies", & MOD. APPROACHES VACCINES: MOL. CHEM. BASIS VIRUS VIRULENCE IMMUNOGENICITY, [PAP. CONF.] 1983 (Publ. 1984), 289-940

Le dossier contient des informations techniques présentées postérieurement au dépôt de la demande et ne figurant pas dans le présent fascicule.

(56) Documents cités:
BIOLOGICAL ABSTRACTS/RRM,
vol. 25, no. 58459, Joint meeting of the National Centre of Studies on Rabies and the World Health Organization on animal rabies, 3 - 5 juin 1982, Nancy. FR,
vol. 5, no. 1-3, pages 117-122, Paris, FR; P. COULON et al.: "Avirulent mutants of the CVS strain of rabies virus"idazines", & FARMACO, ED.
SCI. 1982, 37(2), 133-44 000
BIOLOGICAL ABSTRACTS,
vol. 79, 1985, no. 96532, Biological Abstracts, Inc., Philadelphia, PA, US; I. SEIF et al.: "Rabies virulence: effect on pathogenicity and sequence characterization of rabies virus mutations affecting antigenic site III of the glycoprotein", & J.
VIROL. 1985, 53(3), 926-934s"idazines", & FARMACO, ED. SCI. 1982, 37(2), 133-44 000

## Description

La présente invention concerne un nouveau vaccin antirabique.

Le virus rabique est un rhabdovirus constitué de cinq protéines, dont une protéine externe, la glycoprotéine, qui déclenche les réactions immunitaires chez les animaux inoculés. L'injection de la glycoprotéine purifiée protège l'animal contre une surinfection. Les souches du virus rabique les plus utilisées et notamment la souche CVS sont décrites dans "Rabies Viruses" par H.F. Clark et T.J. Wiktor - Strains of Human Viruses édité par Majer et Plotkin [Karger, Bâle, 1972, p. 177-182]. La séquence en acides aminés de la glycoprotéine de la souche CVS a été décrite par Yelverton et al. "Rabies virus gylcoprotein analogs : biosynthesis in Escherichia coli" Science 219, 614-620.

Les vaccins antirabiques actuellement utilisés sont soit des vaccins fabriqués à partir de virus inactivés, soit des vaccins constitués de souches virales dont la virulence a été atténuée.

L'inactivation des virus peut être réalisée par différents procédés, notamment par des procédés chimiques, tels que le traitement au formol ou à la β-propiolactone.

L'inconvénient majeur de ce procédé de fabrication de vaccins est la manipulation de souches virulentes qui nécessite des conditions de mise en oeuvre très strictes et présente des risques de contamination du personnel de fabrication.

L'atténuation de la virulence des souches virales est une technique bien connue ; elle peut être réalisée par exemple par passages successifs des souches virales sur des embryons de poulet. On obtient ainsi des souches moins virulentes mais qui sont également moins protectrices car elles diffèrent trop des souches de virus "des rues".

On a déjà montré que la plupart des mutants de la souche CVS du virus rabique qui sont résistants aux anticorps monoclonaux anti-gylcoprotéine G n° 194-2 et 248-8 du WISTAR INSTITUTE présentent une pathogénéité considérablement réduite. A cet effet, on peut se référer notamment à l'article d P. COULON et al. dans J. Gen. Virol. (1983), 64, 693-696. Tous les mutants avirulents étudiés jusqu'à présent sont de simples mutants thermostables. Les mutants thermosensibles qui ont déjà été isolés sont soit toujours virulents, soit atténués, ces derniers n'ayant pas un pouvoir protecteur suffisant.

Le problème de la réversion d'une souche mutante avirulente vers l'état initial virulent se pose pour l'utilisation de cette souche comme vaccins vivants ou inactivés.

On a maintenant trouvé un double mutant avirulent et thermosensible de la souche CVS du virus rabique, qui peut être utilisé pour la vaccination contre la rage.

Ce double mutant avirulent thermosensible, dénommé ciaprès TAG₁ a été déposé à la Collection Nationale de Cultures de Microorganismes (C.N.C.M.) INSTITUT PASTEUR-FRANCE le 12 avril 1985 sous le n° I-433. Il a été obtenu à partir d'une souche CVS sauvage dont la séquence en acides aminés diffère de celle définie par Yelverton et al. (voir référence citée précédemment) par le remplacement de la thréonine 36 par une isoleucine et de la valine 122 par une leucine.

Le mutant TAG₁ porte deux mutations sur la glycoprotéine, qui entraînent chacune la perte de la virulence pour les animaux adultes. Ces mutations sont :

1) une mutation thermosensible.

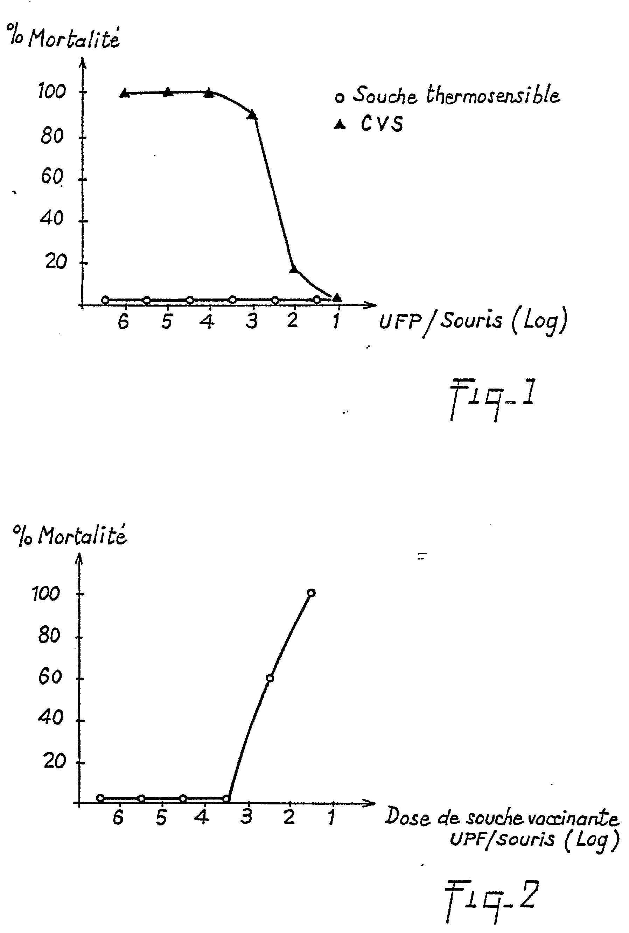

On a trouvé que cette mutation est caractérisée par le remplacement de la leucine 132 par une phénylalanine. Elle entraîne une perte complète du pouvoir pathogène par injection intramusculaire. En effet, la souche portant cette première mutation est avirulente par voie intramusculaire chez la souris adulte comme le montrent les courbes de mortalité de la figure 1 annexée en fonction des doses administrées (unités formant plage). Sur cette figure, la courbe

—o—o—

est relative à la souche mutante et la courbe

—▲—▲—

est relative à la souche CVS d'origine. Les souris inoculées avec cette souche mutante sont protégées contre une surinfection par 10⁷ UFP de CVS par voie intramusculaire comme le montre la courbe de la figure 2 annexée qui est représentative du pouvoir protecteur de cette souche dans ces conditions. Cette souche mutante n'est cependant pas stable. On a en effet noté des réversions vers le phénotype virulent après un passage en cerveaux de souriceaux nouveaux-nés.

2) une seconde mutation caractérisée par le remplacement de l'arginine 333 par une glutamine.

Cette mutation a été sélectionnée à partir de la souche portant la première mutation thermosensible à l'aide des anticorps monoclonaux 248-8 et 21B₄ selon le mode opératoire décrit par I. SEIF et al. dans J. of Virology, Mars 1985, vol 53, p. 926-934.

On sait que cette substitution d'acide aminé entraîne la perte de virulence pour les animaux adultes. Il a aussi été montré que les souches avirulentes portant cette mutation confèrent aux animaux vaccinés une excellente protection contre le virus sauvage injecté par voie intramusculaire [A. FLAMAND et al. "Modern Approaches to Vaccines" Cold Spring Harbor Laboratory 1984, 189-194]. Cependant, ces souches avirulentes présentent l'inconvénient de ne pas être stables, c'est-à-dire de réverser vers l'état virulent initial.

Le double mutant selon l'invention présente la caractéristique spécifique des mutants avirulents et à pouvoir protecteur. Il convient donc bien pour la vaccination des animaux contre la rage.

De plus, c'est un mutant stable. Il a en effet été vérifié par passages successifs en cerveau de souriceaux nouveaux-nés que la conjugaison de ces deux mutations augmentait la stabilité de la souche. En outre, ce mutant $TAG_1$ permet la manipulation de grandes quantités de virus sans danger pour le personnel de fabrication.

Le mutant selon l'invention peut être multiplié sur cellules de rein de hamster nouveaux-nés BHK 21, en présence de milieu GEM (milieu essentiel minimum modification Glasgow commercialisé par FLOW) et de 2 % de sérum de veau à 33°C et en atmosphère humide avec 5% de $CO_2$.

On le titre selon les méthodes habituelles, par exemple par détermination de la dose létale 50 ($DL_{50}$) chez les souriceaux, par immunofluorescence ou détermination des plages formant lyse sous agar.

Il peut être conservé à - 70°C.

Comme tous les mutants avirulents, on notera que le double mutant $TAG_1$ est résistant aux anticorps monoclonaux 248-8 et 194-2 du WISTAR INSTITUTE, par contre il est sensible à l'anticorps monoclonal anti-glycoprotéine $21B_4$ du Laboratoire de Génétique des Virus de Gif-sur-Yvette décrit dans Journal of Virology, Mars 1985, vol.53, p. 926-934.

Pour la vaccination, la dose de mutant à utiliser varie en fonction de l'animal à traiter. A titre indicatif, on précisera que des doses de $10^4$ UFP à $10^7$ UFP/ souris assurent une bonne protection en vaccin vivant.

En vaccin inactivé, le pouvoir vaccinant de $TAG_1$ est comparable à celui de la souche CVS chez la souris. Par le test de Pharmacopée Européenne, on a en effet déterminé que le pouvoir vaccinant de $TAG_1$ est de 1,39 unités internationales par ml (UI/ml) alors que le pouvoir vaccinant de CVS est de 0,95 UI/ml. Ces résultats ont été confirmés par le test de séro-neutralisation selon la Pharmacopée Européenne. Les résultats de ce test, réalisé selon le mode opératoire décrit dans J. Biol. Stand. 1975, 3, p. 365-373, sont consignés dans le tableau ci-après :

| Virus | Dilution (log) | Titre (Unités internationales) |
|---|---|---|
| CVS | − 0,7 | 2,33 |
| | − 1,05 | 2,14 |
| | − 1,40 | 1,03 |
| | − 1,75 | 0,23 |
| | − 2,10 | 0,23 |
| $TAG_1$ | − 0,7 | 2,69 |
| | − 1,05 | 1,22 |
| | − 1,40 | 0,05 |
| | − 1,75 | 0,46 |
| | − 2,10 | 0,05 |

Le mutant selon l'invention peut être administré à titre de vaccin vivant par tous les modes d'administration habituellement utilisés pour la vaccination et notamment par voie intramusculaire. Le mutant est dilué dans un véhicule inerte pharmaceutiquement acceptable, tel que le sérum physiologique.

## Revendications

1. Double mutant avirulent et thermosensible de la souche CVS du virus rabique déposé à la Collection Nationale de Culture de Microorganismes (C.N.C.M.) INSTITUT PASTEUR-FRANCE le 12 avril 1985 sous le n° I-433.

2. Vaccin antirabique, caractérisé en ce qu'il est essentiellement constitué par le double mutant avirulent et thermosensible de la souche du CVS du virus de la rage tel que défini à la revendication 1.

## Claims

1. Avirulent and heat-sensitive double mutant of the CVS strain of the rabies virus deposited at the "Collection Nationale de Culture de Micro-organismes" (C.N.C.M) INSTITUT PASTEUR-FRANCE on April 12, 1985 under No. I-433.

2. Anti-rabies vaccine, characterized in that it is essentially constituted by the avirulent and heat-sensitive double mutant of the CVS strain of the rabies virus such as defined in claim 1.

## Patentansprüche

1. Avirulente und wärmeempfindliche Doppel-Mutante des CVS Stammes des Rabies Virus, welche am 12. April 1985 unter der Nr. I-433 bei der "Collection Nationale de Culture de Micro-organismes" (C.N.C.M) INSTITUT PASTEUR − FRANCE hinterlegt wurde.

2. Anti-Rabies-Impfstoff, dadurch gekennzeichnet, daß er wesentlich aus der avirulenten und wärmeempfindlichen Doppel-Mutante des wie in Anspruch 1 definierten CVS Stammes des Rabies-Virus besteht.

Fig-1

Fig-2